# EUROPÄISCHE PATENTSCHRIFT

(11) **EP 3 285 749 B1**
(45) Veröffentlichungstag und Bekanntmachung des Hinweises auf die Patenterteilung: **05.06.2024**
(21) Anmeldenummer: 16717375.6
(22) Anmeldetag: 20.04.2016
(51) Int. Cl.: A61K 45/06, A61K 9/48, A61K 31/56, A23P 10/30, A23L 33/11, A23L 33/12, A61P 3/02

(54) **GELKAPSEL ENTHALTEND STEROL UND SOLUBILISATOR**
GEL CAPSULE CONTAINING STEROL AND SOLUBILISING AGENT
CAPSULE DE GEL CONTENANT DU STÉROL ET UN AGENT DE SOLUBILISATION

(30) Priorität: 23.04.2015 EP 15164868; 28.04.2015 EP 15165398
(43) Veröffentlichungstag der Anmeldung: 28.02.2018
(73) Patentinhaber: BASF SE, 67056 Ludwigshafen am Rhein (DE)
(72) Erfinder: GIERKE, Juergen, 88489 Wain (DE); WEIDNER, Martin, 36093 Kuenzell (DE); HEER, Marianne, 68623 Lampertheim (DE); SCHMELLER, Thorsten, 67157 Wachenheim (DE); HELGASON, Thrandur, 68163 Mannheim (DE); BOHN, Heribert, 67319 Wattenheim (DE); WEILAND, Anja, 67133 Maxdorf (DE)
(74) Vertreter: BASF IP Association
(86) Internationale Anmeldenummer: PCT/EP2016/058683
(87) Internationale Veröffentlichungsnummer: WO 2016/169941

(56) Entgegenhaltungen:
- EP-A1- 2 042 165
- EP-A1- 2 042 180
- WO-A1-99/56558
- DE-A1- 10 253 111
- US-A1- 2005 153 948
- Anonymous: "VegaPure 95E", internet citation, XP002759247, Gefunden im Internet: URL:http://www.brenntag.com/media/document s/bsi/product_data_sheets/life_science/bas f_plant_sterols/basf_vegapure_95_e_pds.pdf [gefunden am 2016-06-27]
- ACUFF ROBERT V ET AL: "The lipid lowering effect of plant sterol ester capsules in hypercholesterolemic subjects", LIPIDS IN HEALTH AND DISEASE, BIOMED CENTRAL, LONDON, GB, Bd. 6, Nr. 1, 9. April 2007 (2007-04-09), Seite 11, XP021024432, ISSN: 1476-511X, DOI: 10.1186/1476-511X-6-11

## Beschreibung

Die vorliegende Erfindung betrifft Gelkapseln enthaltend Sterol-/Stanolester und Solubilisator zur Verwendung als Nahrungsergänzungsmittel oder als Pharmazeutikum.

Gelkapseln als solches sind bekannt: es gibt sogenannte Hartkapseln und Weichkapseln. Deren Herstellung, die dazu verwendeten Einsatzstoffe wie deren Herstellung ist dem Fachmann hin-länglich bekannt. Die Verwendung von Gelkapseln enthaltend Sterole als Nahrungsergänzungsmittel oder als Pharmazeutikum ist bereits ebenfalls bekannt.

Da Sterole in Wasser nur sehr schlecht löslich sind wird in der Regel ein Öl als Lösemittel verwendet. Sterolester sind besser löslich, sind in der Regel jedoch ebenfalls in Öl oder einer Fett-Substanz gelöst vorliegend.

Da die Sterole und Sterolester demnach in Öl formuliert eingesetzt werden, ist ein signifikanter Anteil der Formulierung ein Öl. Die Menge an Formulierung, die demnach pro Dosiereinheit, also etwa einer Gelkapsel, zugeführt werden kann, ist aber aufgrund der maximal akzeptablen Größe der Gelkapsel limitiert. Wird nur ein Teil dieser Formulierung durch Öl aufgebraucht, so wird die Menge an zuführbarer Sterol oder Sterolestermenge weiter begrenzt. Um die empfohlenen Tagesmengen an freiem Sterol in Höhe von 2 bis 3 g pro Tag und erwachsenem Menschen bereitzustellen, wird angestrebt eine möglichst geringe Anzahl an Gelkapseln pro Tag zuführen zu müssen, wobei die Gelkapseln auch noch möglichst klein sein sollten; dadurch wird zum einen die "Compliance", d.h. das Befolgen der korrekten Dosiermenge durch den Patienten er-höht und damit die Wirksamkeit der Behandlung insgesamt verbessert. Weiterhin wird bei Nahrungsergänzungsmitteln die Akzeptanz durch den Nutzer verbessert und damit letztlich eben-falls die Wirksamkeit der Intervention.

Um die Effektivität der Sterolester-Formulierung zu verbessern müssen die Sterolester und die anderen Inhaltsstoffe eine feinteilige Emulsion bilden, und zwar schon bei niedrigen Scherraten und dem pH-Wert im Magen. Dies wird in der pharmazeutischen Industrie bei pharmazeutischen Formulierungen durch selbst-emulgierende System erreicht. Dort werden allerdings sehr große Mengen an verdünnenden Substanzen wie auch Emulgatoren eingesetzt. Ebenso sind für pharmazeutische Formulierungen eine Vielzahl an Emulgatoren regulatorisch zugelassen, so dass die Anpassung der Formulierung zur Erreichung eines selbst-emulgierenden System relativ gesehen einfacher ist als für Nahrungsmittel. Weiterhin sind die Mengen an pharmazeutisch aktiver Substanz, die in solchen selbstemulgierenden System verabreicht werden sollen, pro Dosis üblicherweise deutlich niedriger als dies für Substanzen im Lebensmittelbereich der Fall ist.

Bei der Verabreichung von Sterolestern pro Dosis liegen die Mengen im Bereich von etwa 1 bis 3 Gramm pro Dosis, weshalb die Gesamtmenge an zu dosierender Formulierung schon sehr groß ist, wenn die Dosis als Kapsel formuliert werden soll. Da im Lebensmittelbereich die Zahl der Emulgatoren wie auch der Tenside (Englisch meist als "surfactants" beschrieben) regulatorisch gesehen stark begrenzt und damit auch die Breite an Eigenschaften der zur Verfügung stehenden Emulgatoren sehr begrenzt ist im Vergleich zu pharmazeutischen Anwendungen, ist das Erreichen eines selbstemulgierenden Systems mit kleinen Mengen Lebensmittel-zugelassenen Emulgatoren und Tensiden eine deutlich größere Herausforderung. Der niedrige pH-Wert im Magen reduziert die Aktivität der anionischen Tenside durch Neutralisation. Daher ist zur Zeit keine effektive Formulierung von Sterolestern in Kapseln verfügbar, die eine ein- oder maximal zweifache Gabe (das heisst die Gabe von ein oder maximal zwei Kapseln pro Tag) ermöglicht, um damit das Tagesmenge an Sterol in Form von Sterolestern zuzuführen.

Daher ist es erstrebenswert, die Formulierung von Sterolen und Sterolestern in Gelkapseln in-soweit zu verbessern, als dass der Gewichtsanteil an Sterol bezogen auf das freie Sterol in ei-ner solchen Formulierung für eine Gelkapsel maximiert wird.

Sterole sind nach bekanntem Wissen unter anderem Phytosterole, das heisst aus Pflanzen gewonnene Sterole.

Sterolester sind zwar prinzipiell alle Ester mit allen denkbaren Carbonsäure. Üblicherweise sind aber im Beriech Nahrung und Gesundheit nur die Ester mit Fettsäuren von Interesse.

Sterolester von Fettsäuren sind herstellbar nach bekanntem Wissen; die am häufigsten angewandte Methode ist die Herstellung aus Sterolen und Fettsäuren durch Veresterung. Der Umsatz und damit auch der reinheitsgrad der Sterolester ist durch die Reaktion nahezu beliebig steuerbar. Angestrebt werden üblicherweise wenigstens 80 Prozent Umsatz zum Sterolester; Umsätze bis nahezu 100 Prozent sind bekannt und technisch durchführbar. Eine höhere Reinheit lässt sich auch erreichen durch Aufreinigung wie Kristallisation, Winterisierung etc. Alle solche Methoden sind hinlänglich bekannt.

Gefunden wurden Formulierungen für Gelkapseln enthaltend im Wesentlichen Sterol-/Stanolester und Emulgator (auch als Solubilisator bezeichnet) gemäß Anspruch 1, sowie Gelkapseln enthaltend diese Formulierungen gemäß Anspruch 5, sowie Verfahren zu ihrer Herstellung gemäß Aspruch 7.

"Im Wesentlichen" bedeutet dabei dass der Gesamtanteil an der Formulierung wenigstens 61 Gewichtsprozent beträgt.

"Solubilisator" bezecihnet im Rahmen dieser Erfindung allgemein Substanzen, die emulgierend und/oder stabilisierend wirken, und werden im Rahmen dieser Erfindung gleichbedeutend auch als "Emulgator" oder Tensid" bezeichnet, wobei bei den erfindungsgemäßen Formulierungen die Kombinationen Lecithin und Polysorbat 80, Lecithin und Zuckerester mit Fettsäuren, oder Lecithin und Ascorbin-Fettsäureester, als Solubilisator ausgewählt sind.

Als Sterolester ist prinzipiell jeder Sterolester aus jedem Sterol verwendbar. Bevorzugt Verwendung finden Sterole aus Pflanzen ("Phytosterole"), die dem Fachmann hinlänglich bekannt sind. Im Rahmen dieser Erfindung sind mit dem Begriff "Sterole" und "Phytosterole" auch deren hydrierte Analoga, die "Stanole", umfasst, sofern nicht explizit anders beschrieben.

Besonders bevorzugt sind die (nicht nachträglich hydrierten) Sterole.

Die Sterole und Stanole, insbesondere Sterole, sind bevorzugt mit Fettsäuren verestert. Als Fettsäure ist prinzipiell jede Fettsäure möglich. Bevorzugt sind Fettsäuren, die aus natürlichem Ursprung gewonnen werden, insbesondere solche aus Pflanzen und marinen Ursprung, oder die diesen jeweils entsprechenden, aber synthetisch hergestellten Fettsäuren. Besonders bevorzugt sind Fettsäuren pflanzlichen oder marinen Ursprungs. Die Fettsäuren können als Reinsubstanz oder als Substanz enthaltend überwiegend eine oder mehrere wenige Fettsäuren eingesetzt werden, oder auch als Gemisch vieler verschiedener Fettsäuren.

Bevorzugt werden Fettsäuren eingesetzt, die einen gesundheitlichen Zusatznutzen haben. Solche Fettsäuren mit gesundheitlichem Zusatznutzen sind insbesondere einfach und mehrfach ungesättigte Fettsäuren wie Omega-3-Fettsäuren, EPA, und DHA; all diese Fettsäuren, deren Herstellung, Gewinnung und Aufreinigung sind dem Fachmann hinlänglich bekannt.

Sterolester im Sinne der vorliegenden Erfindung können auch freie, das heisst nicht veresterte Sterole enthalten. Als Sterolester eingesetzt werden Mischungen aus Sterolester und freien Sterolen, wobei der Sterolestergehalt bevorzugt wenigstens 80, besonders bevorzugt wenigstens 90, ganz besonders bevorzugt wenigstens 95 und insbesondere wenigstens 98 Molprozent (bezogen auf den Sterolanteil im Ester) beträgt sowie alle Werte zwischen diesen Bereichen und bis zu 100 Prozent betragen kann.

Die erfindungsgemäßen Formulierungen für Gelkapseln und die Gelkapseln enthaltend diese Formulierungen enthalten daher bevorzugt Sterolester, insbesondere Sterolester mit einfach oder mehrfach ungesättigten, besonders bevorzugt mehrfach ungesättigten Fettsäuren aus bevorzugt pflanzlichem oder marinem Ursprung, wenigstens einen Solubilisator, bevorzugt aus-gewählt aus Polysorbaten wie Polysorbat 20, 40, 60 und 80, Lecithinen und Sodiumstearoyl-2-lactylate, besonders bevorzugt ausgewählt aus Polysorbat 80 (Polyoxyethylen(20)-sorbitan-monooleat, E433), Lecithin und Sodiumstearoyl-2-lactylat mit einem Veresterungsgrad von 100 bis 140, ganz besonders bevorzugt Polysorbat 80 und/oder Lecithin. Insbesondere bevorzugt werden wenigstens zwei verschiedene Emulgatoren eingesetzt.

Lecithin im Rahmen dieser Erfindung meint eine Zusammensetzung enthaltend einen Anteil an Lecithin bevorzugt gewonnen aus natürlichen Quellen von jeglicher vegetabilen oder tierischen Quelle. Solche Quellen und die Gewinnung von Lecithinen daraus sind dem Fachmann hinlänglich bekannt, etwa aus Eiern, Soja, Sonnenblumen, Raps. Lecithin im Rahmen dieser Erfindung ist bevorzugt eine Mischung enthaltend Lecithinfraktionen, Lysolecithin (wie hydrolysiertes Lecithin, enzymatisch behandeltes Lecithin) und/oder Phospholipide, gewonnen aus jeglichen vegetabilen und/oder tierischen Quellen, bevorzugt vegetabilen Quellen wie Raps, Sonnenblumen und/oder Soja, besonders bevorzugt eine solche aus Sojabohnen gewonnene Mischung, ganz besonders bevorzugt eine solche Mischung enthaltend wenigstens 40, 45, 50, 55, 60, 65, 70 oder 75 oder mehr Prozent an Phosphatidylcholin und wenigstens 3, 4, 5, 6 oder 7% Phosphatidylethanolamin - wobei die Gesamtmenge jeweils 100 Prozent ergibt - , beispielsweise die kommerziellen Produkte der Lecico-Reihe wie die Soja-Lecithine Lecico F 600, Lecico F 580, Lecico F300, Lecico F 200, Lecico F 100, Lecico P900, Lecico P 700, Lecico P 300, die Sonnenblumen-Lecithine Lecico SUN 400, Lecico SUN FM 580, die Raps-Lecithin Lecico RAP 200, der Lipoid- und Phospholidon-Reihen, etwa der , Lipoid P45, Lipoid P75, Lipoid P75-3, Lipoid P100, Lipoid H100, Lipoid R100, Lipoid P100-3, Lipoid S45, Lipoid S75, Phospholidon 80H, Phospholidon 90H, Phospholidon 90G, bevorzugt jeweils mit wenigstens 60, 65, 70 oder 75, 80, 85, 90, 95 oder mehr Prozent an Phosphatidylcholin wie insbesondere Lipoid P75, Lipoid S75, Lipoid P75-3, Lipoid P100-3, Phospholidon 80H, Phospholidon 90H, Phospholidon 90G, ganz besonders bevorzugt solche mit etwa 65 bis 75, insbesondere etwa 70 Prozent Phosphatidylcholin-Anteil.

Die Formulierungen für Gelkapseln und die Gelkapseln enthaltend diese Formulierungen können desweiteren auch Wasser, Alkohole oder deren Mischungen enthalten. Bevorzugt enthalten Formulierungen enthaltend Lecithin als Solubilisator auch Wasser und/oder Alkohole, besonders bevorzugt überwiegend oder ganz überwiegend Alkohole. "Ganz überwiegend" bedeutet dabei, dass die Alkohole noch Restmengen an Wasser enthalten können. Diese Restmengen betragen bevorzugt kleiner 5, kleiner 4, kleiner 3, kleiner 2, kleiner 1 oder gar kleiner 0,5 Prozent Gewichtsanteil im Alkohol.

In einer anderen Ausführungsform enthalten die Formulierungen für die Gelkapseln enthaltend Lecithin als Solubilisator Öle, insbesondere pflanzliche Öle wie Sonnenblumenöl. Die Ölmengen sind aber bevorzugt klein im Vergleich zur Menge an Sterolester und lediglich so groß, um eine einheitliche Lösung des Sterolesters und des Lecithins zu erreichen.

Weitere Inhaltsstoffe sind möglich für die erfindungsgemäßen Formulierungen, verringern aber die Menge an formulierbarem Sterol. Daher sind nur solche Zusätze bevorzugt, die zur Formulierung nötig sind, etwa Stabilisatoren für die Sterolester, wie Antioxidantien, sowie Zuckerester, etwa Mono-, Di, und Tri-ester von Fettsäuren und Zuckern, bevorzugt Sucrose, bevorzugt Monoester von Palmitinsäure und Sucrose, Monoester von Stearinsäure und Sucrose und/oder Monoester von Ölsäure und Sucrose. Übliche und geeignete Antioxidantien sind dem Fachmann hin-länglich bekannt. Die obigen erfindungsgemäßen Formulierungen für Gelkapseln und die Gelkapseln enthaltend diese Formulierungen enthalten daher bevorzugt kleine Mengen an Antioxidantien, bevorzugt ausgewählt aus sterisch gehinderten phenolischen Antioxidantien wie t-Butylhydroxytoluol, t-Butylhydroxyanisol, t-Butylhydroxyquinon; Tocopherole wie alpha, beta, gamma and delta Tocopherol oder Mischungen enthaltend wenigstens zwei dieser Tocopherole; alpha, beta, gamma, und delta Tocotrienole Mischungen enthaltend wenigstens zwei dieser Tocotrienole; Naturstoffextrakte enthaltend wenigstens eine der zuvor genannten Substanzen und/oder phenolische Diterpene wie Carnosol, Carnosolsäure, Polyphenole wie Epigallocatechingallat, Tanninsäure und/oder Isoflavone; insbesondere an Tocopherolen, Ascorbinsäure oder Isoascorbinsäure oder deren geeigneten Derivaten wie Ester mit Fettsäuren, wie Ascorbylpalmitat und Ascorbylstearat. Bevorzugt sind Tocopherole und Ascorbyl-Fettsäureester, insbesondere Tocopherole und Ascorbylpalmitat.

Die erfindungsgemäßen Formulierungen enthalten die folgenden Mengen an Substanzen:
Sterolester:
   wenigstens 60, 65 oder bevorzugt wenigstens 70, besonders bevorzugt wenigstens 75, ganz besonders bevorzugt wenigstens 80, wie insbesondere bevorzugt wenigstens 85 oder gar wenigstens 88 Gewichtsprozent bezogen auf die gesamte Formulierung, die als Füllmaterial für Gelkapseln dient, wie beispielsweise 60, 61, 62, 63, 64, 66, 67, 68, 69, 71, 72, 7,3 74, 76, 77, 78, 79, 81, 82, 83, 84, 86, 87, 89, 90.
Solubilisator:
   für Lecithine und Mischungen enthaltend Lecithinfraktionen und Phospholipide: wenigstens 1, bevorzugt wenigstens 1,1, besonders bevorzugt wenigstens 1,2, ganz besonders bevorzugt wenigstens 1,3 und insbesondere wenigstens 1,4 Gewichtsprozent, als untere Grenze, und bis zu 20 Gewichtsprozent, bevorzugt bis zu 15, besonders bevorzugt bis zu 10 und insbesondere bevorzugt bis zu 5 Gewichtsprozent, als obere Grenze, jeweils bezogen auf die gesamte Formulierung, die als Füllmaterial für Gelkapseln dient, wie beispielsweise 1,5. 1,6, 1,7, 1,8, 1,9, 2,0, 2,1, 2,2, 2,3, 2,4, 2,5, 2,6 2,7, 2,8, 2,9, 3,0, 3,5, 4,0, 4,5, 5,0 und alle Werte dazwischen;
   für Polysorbate wie Polysorbat 80 und Stearoyl-2-lactylate: wenigstens 5, bevorzugt wenigstens 5,5, besonders bevorzugt wenigstens 6,0, ganz besonders bevorzugt wenigstens 6,5 und ins-besondere wenigstens 7,0 Gewichtsprozent bezogen auf die gesamte Formulierung, die als Füllmaterial für Gelkapseln dient, wie beispielsweise 5,1, 5,2, 5,3, 5,4, 5,6, 5,7, 5,8, 5,9, 6,1, 6,2, 6,3, 6,4, 6,6, 6,7, 6,8, 6,9, 7,1, 7,2, 7,3, 7,4, 7,5, 7,6, 7,7, 7,8, 7,9, 8,0, 8,1, 8,2, 8,3, 8,4, 8,6, 8,7, 8,8, 8,9, 9,0, 9,1, 9,2, 9,3, 9,4, 9,5, 9,6, 9,7 9,8, 9,9 10,0, 10,1, 10,2, 10,3, 10,4, 10,5, 10,6, 10,7, 10,8, 10,9, 11,0, 11,5, 12,0, 12,5, 13,0, 13,5, 14,0, 14,5, 15,0, 15,5, 16,0, 16,5, 17,0, 17,5, 18,0, 18,5, 19,0, 19,5, 20,0 und alle Werte dazwischen.
Wasser:
   Wasser kann in Mengen von 0 bis 25 Gewichtsprozent enthalten sein. Bei Verwendung von Polysorbaten als alleinige Solubilisatoren werden Wassermengen von 0 bis 15 Gewichtspro-zent, bevorzugt Null bis 10 Gewichtsprozent bezogen auf die gesamte Formulierung, die als Füllmaterial für Gelkapseln dient, eingesetzt, sowie alle Werte zwischen diesen Endpunkten. "Kleine Menge" bedeutet in diesem Zusammenhang, dass nicht mehr als 10, bevorzugt nicht mehr als 5 Prozent Wasser in der Formulierung vorhanden sind.
   Bei Verwendung von Lecithinen und Mischungen enthaltend Lecithinfraktionen und Phospholipide als alleinige Solubilisatoren werden Wassermengen von 0 bis 25 Gewichtsprozent, bevorzugt 5 bis 23 Gewichtsprozent und besonders bevorzugt von 10 bis 20 Gewichtsprozent bezogen auf die gesamte Formulierung, die als Füllmaterial für Gelkapseln dient, eingesetzt, sowie alle Werte zwischen diesen Endpunkten. "Kleine Menge" bedeutet in diesem Zusammenhang, dass nicht mehr als 20, bevorzugt nicht mehr als 15 Prozent Wasser in der Formulierung vorhanden sind.
Öle:
   Ein Öl oder mehrere Öle kann/können in Mengen von 0 bis 25 Gewichtsprozent enthalten sein, bevorzugt jedoch nicht mehr als 20, besonders bevorzugt nicht mehr als 15, ganz besonders bevorzugt nicht mehr als 10 und insbesondere bevorzugt nicht mehr als 5 Gewichtsprozent bezogen auf die gesamte Formulierung. "Kleine Menge" bedeutet, dass nicht mehr als 10, bevorzugt nicht mehr als 5 Prozent Öl in der Formulierung vorhanden sind.

Geeignete Öle sind prinzipiell alle Öle (d.h. Triglyzeride) natürlichen und synthetischen Ur-sprungs, bevorzugt jedoch natürlichen Ursprungs, insbesondere pflanzlichen oder marinen Ursprungs, ganz insbesondere pflanzlichen Ursprungs, wie Öle von Sonnenblumen, Leinsamen, Flachs, Distel, Mandel, Raps, Kokos, Palm etc. oder der Mischungen, wie dem Fachmann hinlänglich bekannt. Bevorzugt Verwendung finden Öle mit geringen Anteilen an gesättigten Fettsäuren und/oder hohen Anteilen an ungesättigten und insbesondere mehrfach ungesättigten Fettsäuren, da diese einen zusätzlichen gesundheitlichen Vorteil bei der Anwendung bewirken, während gesättigte Fettsäuren und insbesondere Trans-Fettsäuren bevorzugt soweit als technisch möglich und/oder kommerziell (d.h. vor allem kostenseitig) tragbar vermieden werden sollten.

Bevorzugt sind daher Fettsäure-Mischungen, die geringe Mengen an Trans-Fettsäuren, bevorzugt kleiner als 5, 4, 3, 2, oder gar 1 Prozent oder weniger trans-Fettsäuren aufweisen, und/oder, bevorzugt und Mengen an gesättigten Fettsäuren von weniger als 7 Prozent, bevorzugt weniger als 6, 5, 4, 3 oder gar 2 oder 1 Prozent an gesättigten Fettsäuren aufweisen, jeweils bezogen auf die Gesamtmasse an Fettsäuren in der Mischung.

Besonders bevorzugt enthalten die Formulierungen keine Öle.

Als Weitere Inhaltstoffe werden bevorzugt eingesetzt: Ascorbylpalmitat und Zuckerester, jeweils in Mengen von bis zu 20, bevorzugt bis zu 15, besonders bevorzugt bis zu 10 und insbesondere bis zu 5 Gewichtsprozent bezogen auf die auf die gesamte Formulierung, die als Füllmaterial für Gelkapseln dient. Bevorzugt eingesetzt wird jeweils wenigstens 1 Prozent, wie beispielsweise, 1,5, 2, 2,5, 3, 3,5, 4 oder 4,5 Gewichtsprozent.

Die vorliegende Erfindung umfasst daher auch ein Verfahren zur Herstellung einer erfindungsgemäßen Formulierung, bevorzugt zur Herstellung einer der bevorzugten Ausführungsformen der Formulierung wie insbesondere Ausführungsform A und AA.

Ein solches Verfahren umfasst, bevorzugt besteht aus, den folgenden Schritten: a) Zugabe von Sterolester und Solubilisatoren einzeln nacheinander, paarweise, mehrere gleichzeitig oder alle gleichzeitig, bevorzugt alle nacheinander, besonders bevorzugt Zugabe des Sterolesters als erste Substanz und nachfolgende Zugabe der anderen Substanzen vorm, während oder nach dem Erwärmen in Schritt b), besonders bevorzugt vor dem Erwärmen in Schritt b),in ein Mischgefäß; b) Erwärmen der einzeln vorgelegten Substanz oder einer Mischung von mehreren Substanzen auf eine Temperatur oberhalb des Schmelzpunkts des Sterolesters; c) Mischen der erwärmten Mischung bei 500 bis 2500 Upm für eine Dauer von 1 bis 60 Minuten, um eine einheitliche Mischung zu erhalten.

Als Gelkapseln kommen prinzipiell alle Arten von Hart- und Weichkapseln in Frage, hergestellt aus Substanzen natürlichen, etwa pflanzlichen und/oder tierischen Ursprungs, wie auch synthetischen Ursprungs, etwa synthetisch hergestellten oder mittels biotechnologischen Verfahren hergestellten Polymeren. Bevorzugt sind Gelkapseln aus pflanzlichen Substanzen sowie solche aus synthetischen Polymeren.

Solche Materialien, deren Herstellung und Gewinnung sowie deren Verarbeitung zu Gelkapseln ist dem Fachmann hinlänglich bekannt. Grundlegende Übersichten finden sich etwa im Lehrbuch der Pharmazeutischen Technologie, etwa im Kapitel IV, " Die Herstellung von Hart- und Weichgelatinekapseln, Autor: Kurt H. Bauer, Freiburg, Seite 58 bis 82, oder in der 8. Ausgabe dieses Lehrbuches im Kapitel 14, Unterkapitel 6, Seiten 344 bis 355, sowie vielen weiteren Standardlehrbüchern. Hartkapseln beispielsweise zeichnen sich dadurch aus, dass die Kapseln als zweiteilige, zusammengesteckte Leerkapseln produziert werden, die erst nach der Produktion gefüllt und verschlossen werden. Die Hartkapseln werden in den überwiegenden Fällen aus wässriger Lösung im sogenannten Tauchverfahren (S. Stegmann, PZ Prisma, 5, 42-56, 1998) hergestellt. Eine Übersicht zum Stand der Technik des Spritzgiessens zur Herstellung von pharmazeutischen Hartkapseln aus Stärke oder Gelatine ist von L. Eith et al. in Drug Dev. Ind. Pharm., 12, 2113-2126 (1986) gegeben. Die völlig unterschiedlichen Verfahren zur Herstellung von Hart-und Weichgelatinekapseln sind beschrieben in W. Fahrig und U. Hofer, Die Kapsel, Wissenschaftliche Verlagsgesellschaft mbH Stuttgart, 1983, S. 58-82.

Kapseln und deren Herstellung und Verwendung finden sich ebenfalls in vielen Patentschriften. Bekannte Hersteller von Kapseln für vorallem pharmazeutische Anwendungen, Nahrungsmittelergänzungen etc sind die Firmen Catalent (R.P. Scherer ist in Catalent aufgegangen), Banner, Capsugel, Accucaps, Swiss Caps (In Aenova aufgegangen). Von diesen und vielen weiteren Firmen sind entsprechende Veröffentlichungen und Patentanmeldungen und Patente bekannt.

Exemplarisch seien genannt: WO2014/202754 offenbart Weichkapseln mit Acylgellan Gum, Stärke und Plastisierungsmittel; US 20010098784 offenbart Kapseln enthaltend Pflanzenpräparate; US6790495B offenbart die Herstellung von Stärkehaltigen Weichkapseln und eine Apparatur zu deren Herstellung; WO2007/116062 offenbart Kapslen oder Schmelzextrudate enthaltend einen Pflanzenextrakt; US2010291197 offenbart durch Schmelzextrusion hergestellte Kapseln aus Polymermaterial; EP2042180A1 offenbart Weichkapseln enthaltend Phytosterole, 10 bis 100 Prozent Triglyzerid, 1 bis 40 Prozent eine Emulgators mit HLB-Wert größer 12 und 1 bis 80 Prozent eines Co-Emulgators mit HLB-Wert kleiner 11, selbstemulgierendem Füller und frei von Wasser; WO2002074861 offenbart die Verwendung eines Polyols als Gelformer sowie kaltwasserlösliche Gelatinegelkapseln daraus; US2004060258 offenbart die Herstellung von Kapseln, insbesondere Weichkapseln, mittels der Rotary-die-Methode; DE2237545A1 offenbart ein Verfahren zur Herstellung von Mikrokapseln durch Abdampfen einer Polymerlösung in einem organischen Lösemittel; EP1138322A2 offenbart Hartkapseln und EP1136070 (A1) Weichkapseln, jeweils, z.B. für pharmazeutische Anwendungen enthaltend Polymerisate aus Vinylestern und gegebenenfalls weiteren Monomeren, strukturverbessernde Hilfsstoffe sowie weitere übliche Hüllbestandteile, deren Verwendung und Herstellung; EP1926480 (B1) offenbart Weichkapseln und deren Herstellung mit Weichkapselhüllen auf Basis von Polyvinylester-Polyethylenglykol-Pfropfcopolymeren; US55699466 A offenbart Füllungszusanmmensetzungen für elastische Weichkapseln; US5505961A offenbart Gelatinekapseln; US2003085487A offenbart einen Apparat zur Herstellung von Weichkapseln; US2003232076A offenbart kaubare Weichkapseln.

Die vorliegende Erfindung umfasst daher auch ein Verfahren zur Herstellung einer Gelkapsel enthaltend die erfindungsgemäße Formulierung, bevorzugt eine der bevorzugten Ausführungsformen der Formulierung wie insbesondere Ausführungsform A und AA. Als Gelkapselmaterial kommen prinzipiell alle dem Fachmann bekannten Materialien und Herstellverfahren und -schritte in Frage, so lange die Kompatibilität mit der erfindungsgemäßen Formulierung gegeben ist.

Diese Kompatibilität lässt sich leicht beurteilen einerseits anhand der eingesetzten Substanzen für die Formulierung und die Kapselmaterialien, und andererseits durch einfache Versuche mit entsprechenden Kapselmaterialien.

Wie in den Beispielen gezeigt ergaben die Verwendung von einzelnen Emulgatoren keine guten selbstemulgierenden Ergebnisse. Bei Mischung zweier Emulgatoren wurden jedoch sehr gute Ergebnisse und selbstemulgierende System erhalten. Dies war insbesondere überraschend bei SUN FM 580 (Lecithin) und Systerna SP 70 (Zuckerester), das das Systerna-Produkt alleine in der Formulierung unlöslich ist und mittels Rotor-Stator-Mischer dispergiert werden musste. Die Verwendung einer kleinen Menge Systerna-Produkt verbesserte die Selbstemulgierung jedoch drastisch.

Ebenso wurde gefunden, dass die Zugabe eines Ascorbin-Fettsäureesters wie Ascorbylpalmitat und/oder -stearat, insbesondere Ascorbylpalmitat, zu einer Lecithin-enthaltenden Formulierung die selbstemulgierenden Eigenschaften im Magenmodell deutlich verbessert, obwohl Acorbylpalmitat zum einen im Öl bei diesen Temparaturen nicht löslich ist und zum anderen als anionischer Emulgator unter diesen pH-Bedingungen ungeladen ist und daher nur eine sehr geringe Emulsionstabilisierungsaktivität aufweist wie auch eine geringe Löslichkeit im wässrigen Medium. Schließlich wurde ebenso gefunden, dass die Polysorbat-Phase, etwa bei Polysorbat 80, sich aus der Sterolesterphase separiert, dass aber die Zugabe von Lecithin zum System die Separierung verhindert und die selbstemulgierenden Eigenschaften sehr deutlich verbessert im Vergleich zu den jeweils einzeln alleine eingesetzten Substanzen.

In Summe ist festzustellen, dass die Kombination von Ascorbyl-Fettsäureester, bevorzugt Ascorbylpalmitat, sowie Lecithin und Polysorbat, bevozugt Polysorbat 80, besonders gute Eigenschaften wie insbesondere sehr gute selbstemulgierende Eigenschaften einer Sterolesterformulierung enthaltend als weitere Komponente Öl hervorrufen, so dass diese Kombination eine ganz besonders bevorzugte Ausführungsform (Ausführungsform A) der vorliegenden Erfindung ist, wobei die zuvor genannten Mengenverhältnisse (die breiten Bereiche wie auch die jeweils offenbarten engeren und bevorzugten Grenzen, wie insbesondere auch Kombinationen der jeweils bevorzugten Bereiche der verschiedenen Komponenten) einzuhalten und die gegebenenfalls weiteren optionalen Inhaltstoffe enthalten sein können. In einer insbesondere bevorzugten Ausführungsform AA sind keine der optionalen weiteren Inhaltstoffe enthalten.

### Beispiele

### Herstellung der Formulierungen

Phytosterolester und Emulgatoren wurde zusammen gegeben und auf 60 °C erhitzt, um die Kristallisation zu vermeiden und/oder, bevorzugt und die Viskosität zu verringern. Anschließend wurde gemischt (Thinky-Mischer ARE-250 (Thinky Corporation, USA) bei 2000 Upm für 1 min, oder in einem Ultra-turrax Tube drive (IKA, Germany) bei 2000 Upm für etwa 30 min. Der Thinky-Mischer wurde dann verwendet, wenn alle Einsatzstoffe flüssig vorliegen, während der Ultra turrax-Mischer eingesetzt wurde, wenn wenigstens einer der Einsatzstoffe als halbfester oder fester Stoff vorlag und sich nicht leicht in der flüssigen oder bei etwa 60 °C verflüssigten Phytosterolesterphase auflösen ließen (beispielsweise Protein, Ascorbylpalmitat). Die Formulierung wurde auf Raumtemperatur (ca 20 bis 25 °C) gebracht, um über Nacht (das heißt nach etwa 8 bis 12 Stunden) eine eventuelle Schaumbildung bei der Herstellung sich auflösen zu lassen, und untersucht auf Phasenseparation. Zeigte sich keine Phasenseparation, wurden die im Nachfolgenden beschriebenen Anwendungstests durchgeführt.

Alle nachfolgend gezeigten Ergebnisse weisen die Zahlenangaben in Gewichtsprozent auf.

### Anwend u ngstests

### Künstliche Magenlösung:

2 g einer 1 M Salzsäure wurden zu 900ml destilliertem Wasser gegeben, der pH-Wert wurde mit 1M Salzsäure auf 1,6 eingestellt und mit destilliertem Wasser auf 1 Liter aufgefüllt.

### Dünndarm-Lösung ("Small intestine solution"):

0,42g of NaOH-Plätzchen, 3,95 g NaH₂PO₄*H₂O und 6,19 g NaCl wurden in 900 ml destilliertem Wassert aufgelöst. Der pH-Wert wurde mit Natronlauge auf 6,5 eingestellt und die Lösung mit destilliertem Wasser auf 1 Liter aufgefüllt.

### Testung im Magenmodell:

Die Phytosterolester/Emulgator-Formulierung wurde auf etwa 60 °C erhitzt um eine einheitliche flüssige Phase zu erhalten. Dann wurden jeweils 100 ml der Magenlösung und 100ml der Dünndarmlösung auf 37-38 °C erwärmt und jeweils 1 g der Formulierung zugegeben. Die Systeme wurden bei 200 Upm eine Stunde lang gerührt und anschließend visuell begutachtet.

Ergebnisse siehe Tabelle 3.

### CaCo2-Modell

Die Formulierungen wurden ebenfalls im sogenannten Caco2-modell getestet. Dabei wird die Auswirkung der Gabe einer Formulierung auf die Cholesterinaufnahme in Caco2-Zellen geprüft. Dies ist ein Maß, ob und wie gut die Sterolester in der Formulierung die Cholesterinaufnahme der Zellen absenken kann. Je höher die Verhinderung der (d.h. je geringer die) Cholesterinaufnahme ist, desto effektiver ist eine Formulierung.

### Prinzip der Caco2-Testung:

Anzucht der Caco 2 Zellen auf einer porösen Membran; polarisierte Struktur der Zellen, die Nährstoffe (hier: Cholesterin) auf der apikalen Seite aufnehmen und auf der basalen Seite wie-der sezernieren.

Es wird überprüft, inwieweit der Transport von Cholesterin durch die gleichzeitige Supplementierung von Phytosterolen in Kombination mit verschiedenen Vehikeln beeinflusst wird

Schritte der Testung: Anzucht der Zellen; Herstellung der sogenannten "Mixed Micelles"; Bestimmung der Zytotoxizität und Bestimmung der Arbeitskonzentration; Messung derCho-lesterinkonzentration im basalen Kompartiment (6 und 24 Stunden nach Beginn der Supplementation) in 2 Durchgängen, jeweils 3 fache Messung

Zytotoxizitätstest: von allen Testsubstanzen können Konzentrationen von 150µg/ml eingesetzt werden, ohne Beeinträchtigung der Zellen; Arbeitskonzentrationen 50 - 100 - 150 µg / ml wurden ausgewählt

Für die Bildung der Mizellen wurde die eingesetzte Menge Cholesterin für alle Behandlungs-gruppen konstant gehalten. Bei der Analyse der apikal applizierten Lösungen wurde festgestellt, dass sich der Cholesteringehalt in den hergestellten Mizellen unterscheidet (eventuell bedingt durch die gewollte gegenseitige Verdrängung von Cholesterin und Phytosterolen bei der Mizellbildung).

Die Ergebnisse der Cholesterinaufnahme bzw. des Transports durch wurden daher auf den tat-sächlichen Cholesteringehalt der Supplementierungslösungen korrigiert.

### Verwendeter Sterolester:

Vegapure 95 E, der BASF SE, enthält kleine Mengen an verschiedenen Tocopherolen und Ascorbylpalmitate als Antioxidanz; Anteil an Sterolester: mindestens 97 % (Flächenprozent); Anteil an freiem Sterol: bis zu 6 % (Flächenprozent)

### Verwendete Solubilisatoren:

- Polysorbat 20: Polyoxyethylen(20)-sorbitan-monolaurat, E432
- Polysorbat 80: Polyoxyethylen(20)-sorbitan-monooleat, E433, "Tween 80"
- Lipoid P 75: Lecithinfraktion und Phospholipide aus Sojabohnen, enthält ca. 75% phosphatidylcholin, 7% phosphatidylethanolamin
- Prefera SSL 6000: Sodiumstearoyl-2-lactylat mit einem Veresterungsgrad von 100 bis 140
- Lametop P 65: DATEM nach FDA-Spezifikation, (DATEM = Englisch: diacetyl tartaric acid ester of mono- and diglycerides, Deutsch: Diacetylweinsäureester mit Mono- und Diglyzeriden), E472e
- Phosal 40 IP: flüssige Zusammensetzung enthaltend ca 40 Prozent Sojabohnen-Phophatidylcholin und Sonnenblumenöl sowie gemischte Tocopherole
- MCT: Medium Chain Triglycerides d.h. mittelkettige Triglyzeride, ein Öl mit vorwiegend C8 bis C10-Fettsäuren
- Lecico SUN FM 580: Sonnenblumen-Lecithin der Firma Lecico; flüssiges, enzymatisch modifiziertes Sonnenblumen-Lecithin, 56 % AU
- CholestOff: kommerziell erhältliches Vergleichsprodukt der Firma NatureMade, USA; enthaltend pflanzliche Sterole und Stannole.

Die folgende Tabelle 1 (1a und 1b) zeigt getestete Referenzformulierungen.

**Tabelle 1a: Zusammensetzungen**

| | | in Gramm | | | | | |
|---|---|---|---|---|---|---|---|
| Nr. | Vehikel | | Vegapure 95 E | Propylen Glycol | MCT | Wasser | Summe |
| 1 | Polysorbat 20 | 7,5 | 31,5 | 0 | 0 | 0 | 39 |
| 2 | Prefera SSL 6000 | 7,5 | 31,5 | 0 | 0 | 0 | 39 |
| 3 | Polysorbat 80 + Wasser | 3,75 | 31,5 | 0 | 0 | 3,75 | 39 |
| 4 | Polysorbat 80 | 7,5 | 31,5 | 0 | 0 | 0 | 39 |
| 5 | Lametop P 65 + MCT | 3,75 | 31,5 | 0 | 3,75 | 0 | 39 |
| 6 | Lametop P 65 + Propylen glycol | 3,75 | 31,5 | 3,75 | 0 | 0 | 39 |
| 7 | Lametop P 65 | 7,5 | 31,5 | 0 | 0 | 0 | 39 |
| 8 | Phosal 40 IP | 7,5 | 31,5 | 0 | 0 | 0 | 39 |
| 9 | Lipoid P 75 + Wasser | 0,63 | 31,5 | 0 | 0 | 6,87 | 39 |

**Tabelle 1b: Zusammensetzungen (Fortsetzung von Tabelle 1a)**

| | | Gewichtsprozent | | | | | |
|---|---|---|---|---|---|---|---|
| Nr. | Vehikel | Vehikel | Vegapure 95 E | Propylen Glycol | MCT | Wasser | Summe |
| 1 | Polysorbat 20 | 19,23% | 80,77% | 0,00% | 0,00 % | 0,00% | 100,00% |
| 2 | Prefera SSL 6000 | 19,23% | 80,77% | 0,00% | 0,00 % | 0,00% | 100,00% |
| 3 | Polysorbat 80 + Wasser | 9,62% | 80,77% | 0,00% | 0,00 % | 9,62% | 100,00% |
| 4 | Polysorbat 80 | 19,23% | 80,77% | 0,00% | 0,00 % | 0,00% | 100,00% |
| 5 | Lametop P 65 + MCT | 9,62% | 80,77% | 0,00% | 9,62 % | 0,00% | 100,00% |
| 6 | Lametop P 65 + Propylen glycol | 9,62% | 80,77% | 9,62% | 0,00 % | 0,00% | 100,00% |
| 7 | Lametop P 65 | 19,23% | 80,77% | 0,00% | 0,00 % | 0,00% | 100,00% |
| 8 | Phosal 40 IP | 19,23% | 80,77% | 0,00% | 0,00 % | 0,00% | 100,00% |
| 9 | Lipoid P 75 + Wasser | 1,62% | 80,77% | 0,00% | 0,00 % | 17,62% | 100,00% |

### Ergebnisse der Tests:

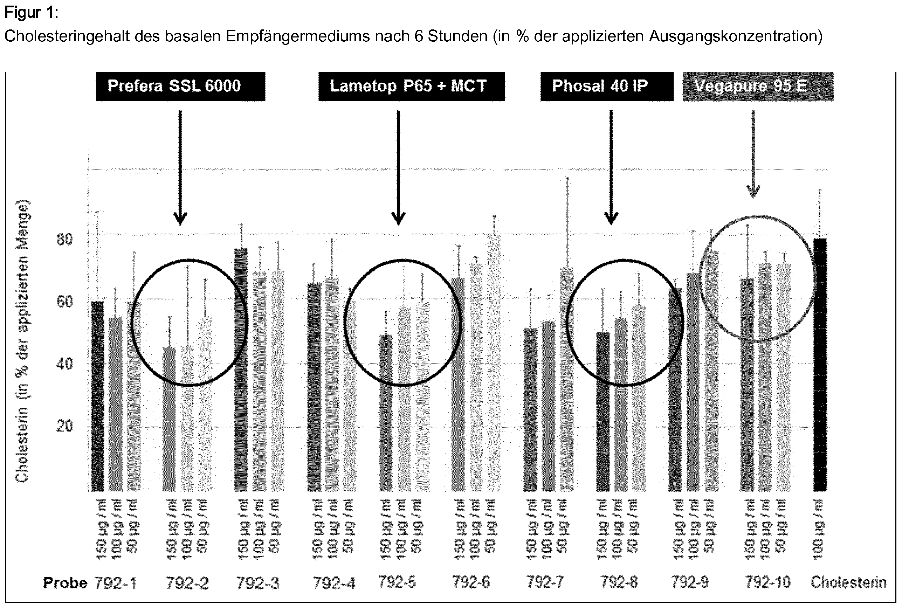

Tabelle 1 (1a und b): getestete Formulierungen - Zusammensetzung
- Figur 1:: Cholesteringehalt des basalen Empfängermediums nach 6 Stunden (in % der applizierten Ausgangskonzentration)

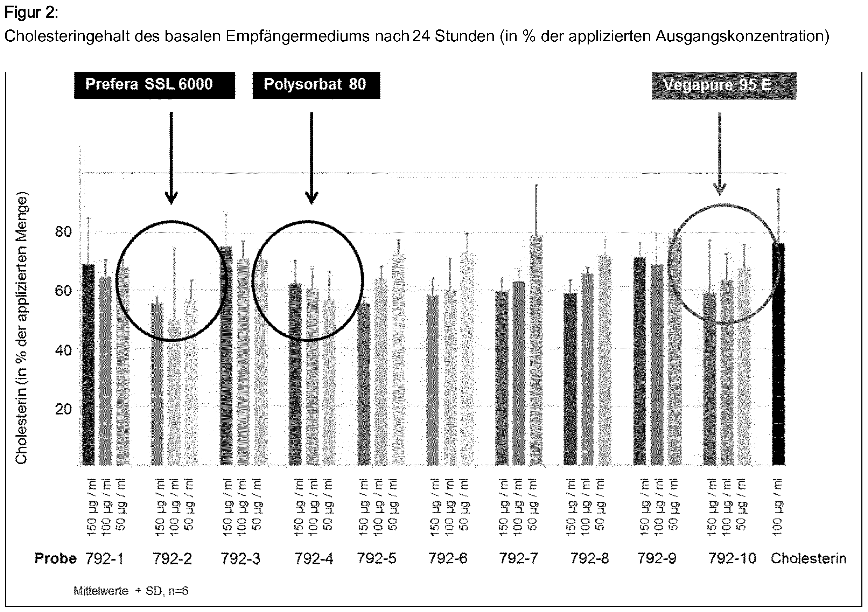

- Figur 2:: Cholesteringehalt des basalen Empfängermediums nach 24 Stunden (in % der applizierten Ausgangskonzentration)
- Figur 3:: Infrarotspektren der Mizellen-Lösungen von Cholesterin ("CHOL") alleine und in Kombination mit den getesteten Formulierungen
- Figur 4:: PCA Analyse der Mizellen Lösungen von Cholesterin (CHOL) alleine und in Kombination mit verschiedenen; Testsubstanzen (C1 bis C10)
- Figur 5:: Ergebnisse zu Tabelle 4

Weitere Ergebnisse aus dem Caco2-Modell (Figur zu Tabelle 3)

| | |
|---|---|
| Tabelle 2: | Beeinflussung des Cholesterintransportes durch die Phytosterol-Vehikel-Kombination im Vergleich zu Vegapure 95E als Einzelsubstanz |
| Tabelle 3: | Weitere Ergebnisse aus dem CaCo2-Modell |
| Tabelle 4: | Ergebnisse der Testung im Magenmodell ("Sterolester" = Vegapure 95 E der BASF) |

**Tabelle 2: Beeinflussung des Cholesterintransportes durch die Phytosterol- Vehikel Kombination (Referenzformulierungen) im Vergleich zu Vegapure 95E als Einzelsubstanz**

| | | Nach 6 Stunden | | Nach 24 Stunden | |
|---|---|---|---|---|---|
| Formulierung enthaltend als Solubilisatoren | Dosierung der Testsubstanz | Cholesterin im Empfangs-kompartiments (% der eingesetzten Menge) | % weniger Cholesterin als Vegapure 95E ohne Vehikel | Cholesterin im Empfangs-kom- partiments (% der eingesetz- ten Menge) | % weniger Cholesterin als Vegapure 95E ohne Vehikel |
| Cholesterin Kontrolle | | 78,72 | | 76,11 | |
| kein Solubilisator (nur Vegapure 95 E) | 50 µg / ml | 70,85 | | 67,62 | |
| | 100 µg / ml | 70,79 | | 63,54 | |
| | 150 µg / ml | 66,32 | | 59,05 | |
| Prefera SSL 6000 | 50 µg / ml | 54,46 | 23% | 56,96 | 16% |
| | 100 µg / ml | 54,46 | 23% | 59,97 | 6% |
| | 150 µg / ml | 45,10 | 32% | 55,46 | 6% |
| Polysorbat 80 | 50 µg / ml | 59,23 | 16% | 56,77 | 16% |
| | 100 µg / ml | 66,56 | 6% | 60,32 | 5% |
| | 150 µg / ml | 65,02 | 2% | 62,11 | n.a. |
| Lametop P65 + MCT | 50 µg / ml | 69,41 | 2% | 72,51 | na. |
| | 100 µg / ml | 53,04 | 25% | 63,92 | n.a. |
| | 150 µg / ml | 50,74 | 23% | 55,64 | 6% |
| Phosal 40 IP | 50 µg / ml | 57,76 | 18% | 71,85 | n.a. |
| | 100 µg / ml | 53,88 | 24% | 65,75 | n.a. |
| | 150 µg / ml | 49,67 | 25% | 58,78 | 0% |

### Messung der Verdrängung von Cholesterin durch Phytosterole bei der Mizellenbildung

Cholesterin und Phytosterole sind schlecht wasserlöslich und werden im Körper zusammen mit Fettsäuren und Gallensalzen in den Mischmizellen transportiert.

Als wichtiger Wirkmechanismus der Phytosterole für die Cholesterinsenkung gilt die Verdrängung von Cholesterin aus den Mizellen.

Ziel der Messung ist die Bestimmung des Einflusses der Testsubstanzen auf die CholesterinKonzentration in der erhaltenen Mizellen-Lösung, um somit einen Hinweis auf die mögliche Verdrängung des Cholesterins aus den Mizellen zu erhalten.

Die Testsubstanzen wurden in 5 verschiedenen Konzentrationen zusammen mit einer definierten Cholesterin-Konzentration zur in-vitro Mizellenbildung eingesetzt.

Die erhaltenen Mizellen-Emulsionen urden Infrarot-spektroskopisch mit dem SpeCCs- Analyzer (Cetics Healthcare) untersucht.

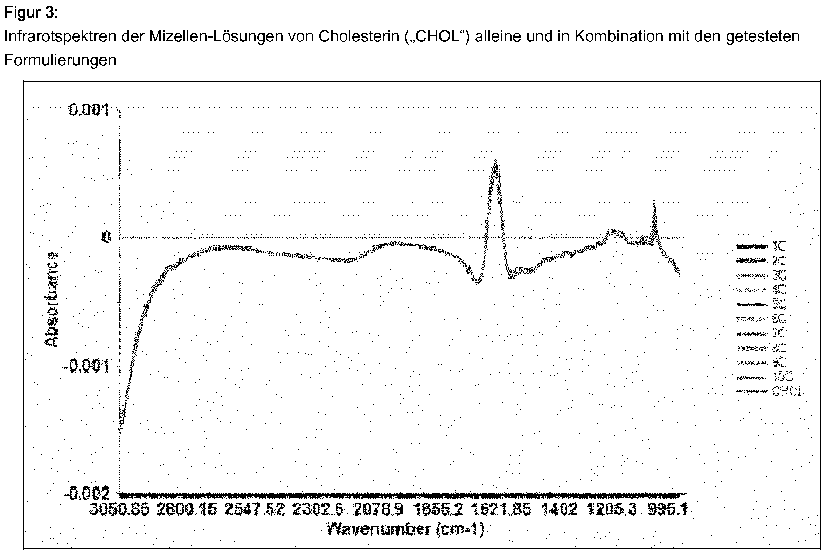

Die Infrarotspektren (Figur 3) unterscheiden sich auf den ersten Blick nur geringfügig aufgrund der Ähnlichkeit in der chemischen Struktur von Cholesterin und Phytosterolen. Die weitere Auswertung erfolgt statistisch mit der Hauptkomponentenanalyse (PC), mit deren Hilfe die Ähnlichkeit von Proben bestimmt werden kann.

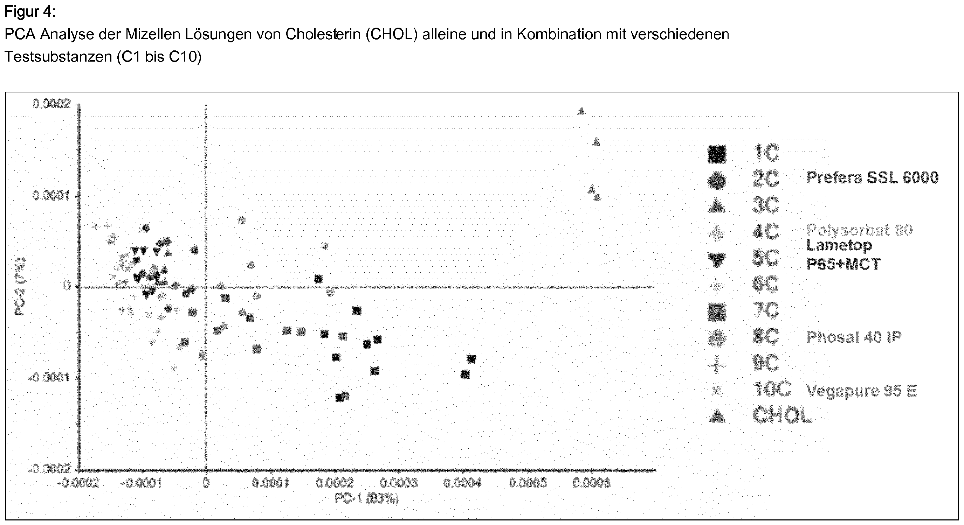

### PCAAnalyse der Mizellen Lösungen von Cholesterin (CHOL) alleine und in Kombination mit verschiedenen Testsubstanzen (C1 bis C10) (Figur 4)

Ergebnis: Die Formulierung mit Lipoid P75 + Wasser unterscheidet sich am deutlichsten von den reinen Cholesterin-Mizellen, gefolgt von Vegapure 95 E, Polysorbat 80, Lametop P65+ MCT und Prefera SSL 6000, was als Maß für die Verdrängung von Cholesterin aus den Mizellen gewertet werden kann.

**Tabelle 3: CaCo 2 - Modell Zwischenergebnisse - Beeinflussung des Cholesterintransports durch Kombinationen aus Phytosterolestern und Emulgatoren (die in den Versuchen 88 und 89 verwendeten Formulierungen sowie CholestOff sind Referenzformulierungen)**

| Getestete Formulierungen | Dosierung der Testsubstanz (µg / ml) | Cholesterin-kon- zentration im Empfangsme- dium in µg / ml (nach 6 Stun- den) | Cholesterin-konzentration im Empfangsmedium in µg / ml (nach 24 Stunden) |
|---|---|---|---|
| Versuch 88; Vegapure 95 E (ohne Emulgator) | 150 | 69,9 | 64,9 |
| Versuch 88; Vegapure 95 E (ohne Emulgator) | 100 | 65,0 | 64,7 |
| Versuch 88; Vegapure 95 E (ohne Emulgator) | 50 | 68,2 | 64,4 |
| Versuch 89: Vegapure 95 E mit Lipoid 75 und Sonnenblumenöl | 150 | 63,2 | 63,3 |
| Versuch 89: Vegapure 95 E mit Lipoid 75 und Sonnenblumenöl | 100 | 65,7 | 61,0 |
| Versuch 89: Vegapure 95 E mit Lipoid 75 und Sonnenblumenöl | 50 | 62,7 | 59,4 |
| Versuch 100: Vegapure 95 E und Lecico SUN FM 580 und Tween 80 | 150 | 61,3 | 57,4 |
| Versuch 100: Vegapure 95 E und Lecico SUN FM 580 und Tween 80 | 100 | 60,6 | 60,1 |
| Versuch 100: Vegapure 95 E und Lecico SUN FM 580 und Tween 80 | 50 | 57,2 | 58,6 |
| CholestOff (kommerziell erhältliches Vergleichsprodukt) | 150 | 59,42 | 60,4 |
| CholestOff (kommerziell erhältliches Vergleichsprodukt) | 100 | 56,93 | 58,3 |
| CholestOff (kommerziell erhältliches Vergleichsprodukt) | 50 | 60,66 | 60,5 |

Die Ergebnisse in Tabelle 3 zeigen, dass der Cholesterintransport bei Verwendung einer Kombination aus Phytosterolester und Emulgator/en stärker beeinflusst werden kann als durch den Phytosterolester alleine.

Tabelle 4 zeigt die Bewertung der Testung der Formulierungen aus Tabelle 3.

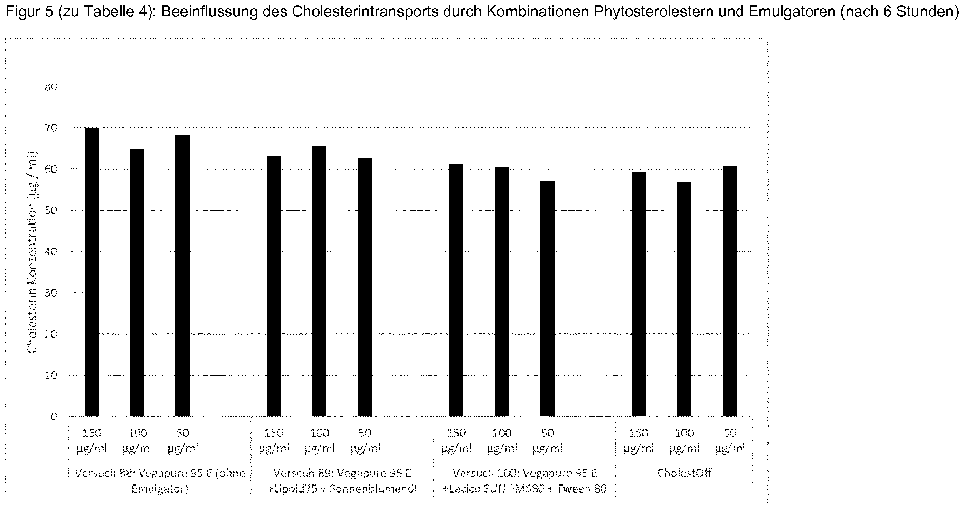

Figur 5 zeigt ausgewählte Ergebnisse im CaCo2-Test von Formulierungen aus Tabelle 3.

Auswertung zu Tabelle 4: **Visuelle Bewertung: Maßstab**
1 = Homogen, kein Öl auf der Oberfläche
2 = weitgehend homogen mit kleinen bis mittleren Öltröpfchen auf der Oberfläche
3 = mittlere Trübung, bemerkbare Ölschicht auf der Oberfläche
4 = leicht weisslich-trübe Erscheinung, dicke Ölschicht auf der Oberfläche
5 = keine Emulgierwirkung, d.h. nahezu vollständige Phasenseparation

**Tabelle 4: Ergebnisse der Testung im Magenmodell ("Sterolester = Vegapure 95 E der BASF); siehe auch Figur 5 (Versuche 88-97 sind Versuche mit Referenzformulierungen)**

| Versuchsnummer | Sterolesterkonzentration (Gew.%) | Einsatzstoff 1 | Einsatzstoff 1 Konz. (Gew.%) | Einsatzstoff 2 | Einsatzstoff 2 Konz (Gew.%) | Einsatzstoff 3 | Einsatzstoff 3 Konz (Gew.%) |
|---|---|---|---|---|---|---|---|
| 88 | 100 | Vegapure 95E | 100 | | | | |
| 89 | 80 | P75 | 1,6 | Sunflower oil | 17,6 | | |
| 90 | 80 | Lecico SUN FM 580 | 20 | | | | |
| 91 | 85 | Lecico SUN FM 580 | 15 | | | | |
| 92 | 90 | Lecico SUN FM 580 | 10 | | | | |
| 93 | 80 | Tween 80 | 20 | | | | |
| 94 | 98 | Tween 80 | 2 | | | | |
| 95 | 90 | Ascorbyl palmitate | 10 | | | | |
| 96 | 98 | Ascorbyl palmitate | 2 | | | | |
| 97 | 80 | Lecico SUN 400 | 20 | | | | |
| 98 | 80 | Lecico SUN FM 580 | 19 | Tween 80 | 1 | | |
| 99 | 80 | Lecico SUN FM 580 | 18 | Tween 80 | 2 | | |
| 100 | 80 | Lecico SUN FM 580 | 16 | Tween 80 | 4 | | |
| 101 | 80 | Lecico SUN FM 580 | 18 | Ascorbyl palmitate | 2 | | |
| 102 | 80 | Lecico SUN FM 580 | 16 | Ascorbyl palmitate | 4 | | |
| 103 | 80 | Lecico SUN FM 580 | 12 | Ascorbyl palmitate | 8 | | |
| 104 | 80 | Lecico SUN FM 580 | 8 | Ascorbyl palmitate | 12 | | |
| 105 | 85 | Lecico SUN FM 580 | 13,5 | Ascorbyl palmitate | 1,5 | | |
| 106 | 90 | Lecico SUN FM 580 | 9 | Ascorbyl palmitate | 1 | | |
| 107 | 80 | Lecico SUN FM 580 | 16 | Ascorbyl palmitate | 2 | Tween 80 | 2 |
| 108 | 80 | Lecico SUN FM 580 | 19 | Systerna SP70 | 1 | | |
| 109 | 80 | Lecico SUN FM 580 | 18 | Systerna SP70 | 2 | | |

**Tabelle 4 - Fortsetzung: Ergebnisse der Testung im Magenmodell ("Sterolester = Vegapure 95 E der BASF); siehe auch Figur 5**

| Versuchsnummer | Magenlösung: Trübung (NTU) | Magenlösung: Visuelle Bew-ertung | Darmlösung: Trübung (NTU) | Darmlösung: Visuelle Bewertung |
|---|---|---|---|---|
| 88 | 0,3 | 5 | 1 | 5 |
| 89 | 485 | 2 | 317 | 3 |
| 90 | 266 | 3 | 1000 | 2 |
| 91 | 59 | 4 | 763 | 3 |
| 92 | 71 | 4 | 492 | 4 |
| 93 | 63 | 4 | 111 | 4 |
| 94 | 102 | 3 | 27 | 4 |
| 95 | 9,2 | 4 | 1000 | 2 |
| 96 | 1,1 | 5 | 90 | 4 |
| 97 | 31 | 4 | 814 | 2 |
| 98 | 554 | 2 | 1000 | 1 |
| 99 | 1000 | 1 | 1000 | 1 |
| 100 | 769 | 2 | 1000 | 1 |
| 101 | 25 | 4 | 922 | 2 |
| 102 | 237 | 2 | 46 | 5 |
| 103 | 516 | 2 | 50 | 5 |
| 104 | 1000 | 3 | 1000 | 1 |
| 105 | 43 | 4 | 679 | 2 |
| 106 | 18 | 5 | 379 | 3 |
| 107 | 383 | 2 | 1000 | 2 |
| 108 | 375 | 2 | 830 | 2 |
| 109 | 51 | 4 | 539 | 3 |

## Patentansprüche

1. Formulierung für Gelkapseln enthaltend Sterol-/Stanolester und Solubilisator, wobei mindestens 61 Gewichtsprozent der Formulierung aus Sterol-/Stanolester und Solubilisator besteht, wobei als Solubilisator die Kombinationen Lecithin und Polysorbat 80, Lecithin und Zuckerester mit Fettsäuren, oder Lecithin und Ascorbin-Fettsäureesters, ausgewählt sind.

2. Formulierung nach Anspruch 1, wobei als Solubilisatoren Lecithin und Ascorbyl-Fettsäureester, bevorzugt Ascorbylpalmitat, ausgewählt sind.

3. Formulierung nach einem der Ansprüche 1 bis 2, wobei die Formulierung neben Sterol-/Stanolester und Solubilisatoren nur noch Wasser und/oder Öl in Mengen von jeweils unabhängig voneinander bis zu 10 Gewichtsprozent bezogen auf die Formulierung enthält.

4. Formulierung nach einem der vorigen Ansprüche, wobei die Fettsäuren im Sterol-/Stanolester Omega-3-Fettsäuren wie EPA und/oder DHA enthalten in einer Menge von wenigstens 30 Gewichtsprozent bezogen auf den Fettsäureanteil.

5. Gelkapsel enthaltend eine Formulierung nach einem der Ansprüche 1 bis 4 in Form einer Weich- oder Hartkapsel.

6. Gelkapsel nach Anspruch 5 zur Verwendung als Nahrungsergänzungsmittel oder als Pharmazeutikum.

7. Verfahren zur Herstellung einer Formulierung nach einem der Ansprüche 1 bis 4, **dadurch gekennzeichnet dass** das Verfahren die folgenden Schritte enthält, bevorzugt nur aus den folgenden Schritten besteht:
a. Zugabe von Sterol-/Stanolester und Solubilisatoren einzeln nacheinander, paarweise, mehrere gleichzeitig oder alle gleichzeitig, bevorzugt alle nacheinander, besonders bevorzugt Zugabe des Sterol-/Stanolesters als erste Substanz und nachfolgende Zugabe der anderen Substanzen vor, während oder nach dem Erwärmen in Schritt b), besonders bevorzugt vor dem Erwärmen in Schritt b), in ein Mischgefäß;
b. Erwärmen der einzeln vorgelegten Substanz oder einer Mischung von mehreren Substanzen auf eine Temperatur oberhalb des Schmelzpunkts des Sterol-/Stanolesters und gegebenenfalls der weiteren Zugabe von Substanzen gemäß Schritt a) während dem fortdauernden Erwärmen;
c. Mischen der erwärmten Mischung bei 500 bis 2500 Umdrehungen pro Minute für eine Dauer von 1 bis 60 Minuten, um eine einheitliche Mischung zu erhalten.

## Claims

1. A formulation for gel capsules containing sterol/stanol ester and solubilizer, wherein at least 61 percent by weight of the formulation consists of sterol/stanol ester and solubilizer, wherein the combinations of lecithin and polysorbate 80, lecithin and sugar esters of fatty acids, or lecithin and ascorbyl fatty acid ester, are selected as solubilizer.

2. The formulation according to claim 1, wherein lecithin and ascorbyl fatty acid ester, preferably ascorbyl palmitate, are selected as solubilizers.

3. The formulation according to either of claims 1 and 2, wherein the formulation contains, in addition to sterol/stanol ester and solubilizers, only water and/or oil in amounts of up to 10 percent by weight in each case and independently of one another, based on the formulation.

4. The formulation according to any of the preceding claims, wherein the fatty acids in the sterol/stanol ester contain omega-3 fatty acids such as EPA and/or DHA in an amount of at least 30 percent by weight, based on the fatty acid component.

5. A gel capsule containing a formulation according to any of claims 1 to 4 in the form of a soft capsule or hard capsule.

6. The gel capsule according to claim 5 for use as a food supplement or as a pharmaceutical.

7. A process for producing a formulation according to any of claims 1 to 4, **characterized in that** the process comprises the following steps, preferably only consists of the following steps:
a. addition of sterol/stanol ester and solubilizers individually in succession, in pairs, several at the same time or all at the same time, preferably all in succession, particularly preferably addition of the sterol/stanol ester as a first substance and subsequent addition of the other substances before, during or after the heating in step b), particularly preferably before the heating in step b), to a mixing vessel;
b. heating of the individually charged substance or a mixture of multiple substances to a temperature above the melting point of the sterol/stanol ester and if applicable the further addition of substances according to step a) while continuously heating;
c. mixing of the heated mixture at 500 to 2500 revolutions per minute for a period of 1 to 60 minutes to obtain a homogeneous mixture.

## Revendications

1. Formulation pour des capsules de gel contenant de l'ester de stérol/stanol et un agent de solubilisation, au moins 61% en poids de la formulation étant constituée par l'ester de stérol/stanol et l'agent de solubilisation, les combinaisons lécithine et Polysorbate 80, lécithine et ester de sucre avec des acides gras ou lécithine et ester d'acide gras d'ascorbine étant choisies comme agent de solubilisation.

2. Formulation selon la revendication 1, la lécithine et l'ester gras d'ascorbyle, de préférence le palmitate d'ascorbyle, étant choisis comme agents de solubilisation.

3. Formulation selon l'une des revendications 1 à 2, la formulation contenant, outre l'ester de stérol/stanol et les agents de solubilisation, uniquement encore de l'eau et/ou de l'huile en une quantité, à chaque fois indépendamment l'une de l'autre, de jusqu'à 10% en poids par rapport à la formulation.

4. Formulation selon l'une des revendications précédentes, les acides gras dans l'ester de stérol/stanol contenant des acides oméga-3, tels que l'EPA et/ou le DHA en une quantité d'au moins 30% en poids par rapport à la proportion d'acide gras.

5. Capsule de gel contenant une formulation selon l'une des revendications 1 à 4 sous forme d'une capsule molle ou d'une capsule dure.

6. Capsule de gel selon la revendication 5 destinée à être utilisée comme complément alimentaire ou comme agent pharmaceutique.

7. Procédé de préparation d'une formulation selon l'une des revendications 1 à 4, **caractérisé en ce que** le procédé contient les étapes suivantes et est de préférence uniquement constitué par les étapes suivantes :
a. ajout d'ester de stérol/stanol et d'agents de solubilisation, individuellement les uns après les autres, par paires, plusieurs en même temps ou tous en même temps, de préférence tous en même temps, de manière particulièrement préférée ajout de l'ester de stérol/stanol comme première substance et ajout consécutif des autres substances, avant, pendant ou après le chauffage dans l'étape b), de préférence avant le chauffage dans l'étape b), dans un récipient de mélange ;
b. chauffage de la substance unique disposée au préalable ou d'un mélange de plusieurs substances à une température supérieure au point de fusion de l'ester de stérol/stanol et le cas échéant ajout supplémentaire de substances selon l'étape a) pendant la poursuite du chauffage ;
c. agitation du mélange chauffé à 500 jusqu'à 2500 tr/min pendant une durée de 1 à 60 minutes pour obtenir un mélange uniforme.
